# EUROPEAN PATENT APPLICATION

(11) **EP 0 807 450 A1**
(43) Date of publication of application: **19.11.1997**
(21) Application number: 97303355.8
(22) Date of filing: 16.05.1997
(51) Int. Cl.: A61M 25/02, A61M 1/00

(54) **Surgical devices**

(30) Priority: 18.05.1996 GB 9610461
(71) Applicant: Redmond, Anthony Damien, Sale, Greater Manchester M33 6GA (GB)
(72) Inventor: Redmond, Anthony Damien, Sale, Greater Manchester M33 6GA (GB)
(74) Representative: Yelland, William Alan

(57) **Abstract**

A thoracic and surgical drainage tubing securing device of a king comprising a plastics member (10) which can be sutured to the skin and has a central hole (11) through which tubing (15) can be passed; the plastics member is at least partially flexible and comprises a plurality of lugs (13,14) around the hole (11) which lugs (14) can be bent so that one or a plurality thereof can be sutured to the skin (20) and another or a plurality thereof can be brought into confrontation with the tube (15) and secured tightly around the tube by means of thread, adhesive tape and/or a bonding adhesive to bond the lug or lugs to the tube externally of the point of entry of the tube into the person's body.

## Description

This invention concerns surgical devices for use in securing thoracic and surgical drainage tubing to the skin of a person.

There is a well recognised problem in securing surgical drainage tubing to the skin.

The consequences of tubes becoming misplaced are serious and in the case of thoracic drainage tubes can be life threatening.

The tubes are required to be smooth and pliable making it difficult to secure them to the skin with surgical thread.

The tubes may be in place for several days. During this time the patient may be mobile.

A smooth round tube running through moist tissue is inherently insecure and this is exacerbated obviously by patient movement. However patients need to be as mobile as possible to prevent post operative blood clots.

Thoracic drainage tubing must not be penetrated as a seal must be maintained to prevent air leaking back into the thorax. This prohibits transfixtion of the tubing with a thread through the tube itself.

Thoracic drainage tubes may also need repositioning making it essential that any securing device is not permanent when first applied.

Any method of securing the tubes must also allow easy observation of the tube to ensure it remains in place and is functioning.

In a different field, namely relating to peritoneal dialysis tubes, an eyelet device has been proposed, which device can be sutured to the skin of a patient in order to serve as a guide for a dialysis tube. The eyelet comprises a rigid plastic disc with a central hole through which the tube can slide, and peripheral holes to receive the sutures.

This known device does not solve the above problems and therefore the traditional methods of attempting to secure drainage tubing remain in use.

One such method is to place a series of sutures in the skin and to wrap the suture thread around the tubing before it is tied tightly to the tubing. Inevitably the thread loosens and unravels around the tubing, particularly when the patient moves.

Also, surgical adhesive tape is often used in addition to such a thread. This has to be wrapped around the tube and then applied to the skin. The use of such a tape has the disadvantages that it obscures the tube from view, cannot be re-sited and loosens with body sweat and movement over a matter of hours.

In order to enable the above problems to be solved the present invention provides a thoracic and surgical drainage tubing securing device of a kind comprising a plastics member which can be sutured to the skin and has a central hole through which tubing can be passed which is characterised in that the plastics member is at least partially flexible and comprises a plurality of lugs around the hole which lugs can be bent so that one or a plurality thereof can be sutured to the skin and another or a plurality thereof can be brought into confrontation with the tube and secured tightly around the tube by means of thread, adhesive tape and/or a bonding adhesive to bond the lug or lugs to the tube externally of the point of entry of the tube into the person's body.

The lugs need not be symmetrical, but are conveniently so to minimise the risk of mis-selection during emergency procedures. The minimum number of such lugs is preferably at least four, with at least two diametrically opposed ones thereof forming a first array, and at least another two thereof forming a second array.

The face of the member intended to abut the person's skin is preferably smooth and planar for the comfort and wellbeing of the patient.

The opposite surface of the member may be ribbed or otherwise profiled to increase the security with which the tube is gripped.

Where adhesive is used to secure the lugs to the tubing, said adhesive may be pre-applied to the lugs of the member and covered with a peel-off tape prior to the lugs being brought into engagement with the tube.

The device may include a tubular extension coaxial with the hole.

Said extension may be intended to extend through the body wall of a person to be secured in place by the one array being secured to the skin of the person, so as to be capable of receiving an insert tube which can be secured to the device by the lugs of the second array.

Alternatively, the extension may be intended to project from the surface of the skin of a person, whereby to receive a connecting tube, or to have a drainage tube inserted through the extension and through the body wall of the person, and in which case all the lugs may be sutured to the skin of the person, and the inserted tube and extension secured together by means of surgical tape wrapped around the junction therebetween.

The device is preferably manufactured from a plastics material similar to or the same as the plastics materials usually used for drainage tubes.

The invention will be described further, by way of example, with reference to the accompanying diagrammatical drawings, wherein:-
FIGURE 1 shows a device of the invention in front elevation;
FIGURE 2 is a perspective view showing the device in an in- use condition sutured to the skin of a person; and
FIGURE 3 is a perspective view showing a modified device of the invention.

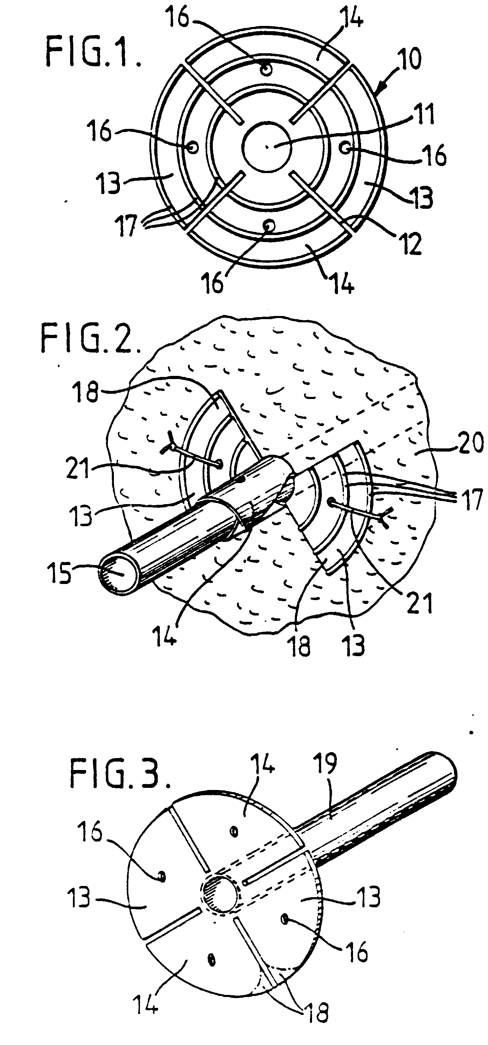

The device shown in FIGURES 1 and 2 comprises a flexible plastics disc 10 having a central hole 11, and slots 12 extending radially from adjacent the holes between radially extending lugs 13 and 14. In this particular example there are four lugs of symmetrical form which are notionally divided into a first array comprising the diametrically opposed lugs 13 and a second array comprising the diametrically opposed lugs 14. In use, the disc is passed over a tube 15 leading through the skin into a person's body until it abuts the skin 20. The lugs 13 of the first array are then sutured by stitches 21 through lug apertures 16 to the skin, and the lugs 14 of the second array are folded forwards and wrapped around the tube 15 before being tied, bound or adhesively secured to the tube so as to be fixed to the tube to secure the tube in position.

The front face of the disc is provided with ribs 17 to enhance the grip on the tube, whereas the reverse face (not shown) is smooth and planar to minimise irritation to the patient and the patient's skin.

In the modified embodiment shown in FIGURE 3 the device has a central tubular extension 19 coaxial with the hole and of the same internal bore diameter as the hole. This tubular extension can be passed through the skin into the person's body so as to serve as a drain or as a guide for insertion of a drainage tubing. Again the lugs of one array are securable to the patient's skin and the lugs of the other array are intended to be secured to the drainage tube so as to locate the drainage tube in position.

As is evident from FIGURE 2, in all embodiments the flexible root portions of the lugs attached to an annular portion of the disc immediately surrounding the aperture 11 serve as hinges about which the lugs can be bent quite abruptly so as to bring the remainder of the lugs alongside the drainage tubes.

The invention is not confined to details of the foregoing examples and many variations are possible within the scope of the invention. For example the number and shapes of the lugs may be determined to suit any particular needs. In the minimum case only two lugs are required, one to be attached to the skin and the other to be attached to the drainage tube. However, such a very simple form of device could well lead to some instability even if the lug attached to the skin was provided with a plurality of holes 16 so that it could be attached by a plurality of sutures to the skin. Equally each array could comprise three lugs, preferably arranged equidistantly around the hole. In order to avoid overlapping of the lugs around the tube, the corner portions 18 of the lugs could be cut away so that the whole of the front surface of each lug abuts the drainage tube to avoid overlapping of the lugs. The removal of the corners of the lugs could well also increase patient comfort, i.e. the corner portions are preferably rounded so as to present a smooth contour to the skin.

The lugs of one array may be of different shape and size to the lugs of the other array, e.g. the lugs intended for embracing of the tube 15 could well be longer than the lugs intended to be attached to the skin.

The lugs, or at least the lugs intended to be attached to the drainage tube could be provided with a bonded-on adhesive layer, normally covered by a peel-off strip of sheet material to expose one surface of the adhesive for attachment to the tube.

It will be readily appreciated that the surgeon has several options to secure the tube, e.g.
1. to pass thread through the apertures in the lugs so as to pull the lugs of the disc close against the tube, and wrapping the thread around the tube and lugs 14 several times before tying the thread securely;
2. to wrap a narrow band of adhesive tape around the lugs 14 and tubing 15;
3. permanent security can be achieved by the application of clinical glue to the lugs 14 and tube 15.

The methods 1 and 2 above allow the tube 15 to be removed by cutting the thread or the adhesive tape. In all embodiments the device and the tube may be removed by cutting the sutures 21 holding the device to the skin.

The device of the invention offers many advantages, in particular it:
1. Fixes the tube to the skin securely yet allows the tube to remain clearly visible at all times and the patient to remain fully mobile.
2. Allows the tube to be re-sited as many times as required while keeping the tube secure.
3. Offers a method of absolute security when the lugs of the disc are glued to the tube and the disc stitched to the skin.

Even without the glue there is no other device or method that gives this degree of security yet allows the tube to be removed so easily. Simply cutting the sutures that tether the device to the skin allows the tube to be withdrawn in the usual fashion.

The device does not alter the standard method of inserting chest drains or other surgical tubing. No new surgical techniques are required.

The device can be used by all those already trained to insert surgical drainage tubes without additional training or experience. Simple written instructions will allow the operator to use the device.

The invention also provides and includes a device for securing a surgical drainage tube to a person's skin having any novel feature or combination of features disclosed herein or in the accompanying drawing, and any mechanical or functional equivalent of any of such features.

## Claims

1. A thoracic and surgical drainage tubing securing device of a kind comprising a plastics member (10) which can be sutured to the skin and has a central hole (11) through which tubing (15) can be passed, which is characterised in that the plastics member is at least partially flexible and comprises a plurality of lugs (13,14) around the hole (11) which lugs (14) can be bent so that one or a plurality thereof can be sutured to the skin (20) and another or a plurality thereof can be brought into confrontation with the tube (15) and secured tightly around the tube by means of thread, adhesive tape and/or a bonding adhesive to bond the lug or lugs to the tube externally of the point of entry of the tube into the person's body.

2. A device as claimed in Claim 1 wherein the lugs (13,14) are symmetrical to minimise the risk of mis-selection during emergency procedures.

3. A device as claimed in Claim 1 wherein the number of such lugs is at least four, with at least two diametrically opposed ones (13) thereof forming a first array, and at least another two (14) thereof forming a second array.

4. A device as claimed in Claim 1 wherein the face of the member intended to abut the person's skin is smooth and planar for the comfort and wellbeing of the patient.

5. A device as claimed in Claim 5 wherein the opposite surface (18) of the member is ribbed or otherwise profiled to increase the security with which the tube is gripped.

6. A device as claimed in Claim 1 wherein adhesive is used to secure the lugs (14) to the tubing; said adhesive being pre-applied to the lugs of the member and covered with a peel-off tape prior to the lugs being brought into engagement with the tube.

7. A device as claimed in Claim 1 wherein the device includes a tubular extension (19) coaxial with the hole.

8. A device as claimed in Claim 7 as appended to Claim 3 and wherein said extension (19) is intended to extend through the body wall of a person to be secured in place by the one array being secured to the skin of the person, so as to be capable of receiving an insert tube (15) which can be secured to the device by the lugs (14) of the second array.

9. A device as claimed in Claim 7 wherein the extension (19) is intended to project from the surface of the skin of a person, whereby to receive a connecting tube (15), or to have a drainage tube (15) inserted through the extension and through the body wall of the person, and wherein all the lugs (13,14) are sutured to the skin of the person, and the received or inserted tube (15) and extension (19) are secured together by means of surgical tape wrapped around the junction therebetween.

10. A device as claimed in Claim 1 wherein the device is manufactured from a plastics material similar to or the same as the plastics materials usually used for drainage tubes.
